# EUROPÄISCHE PATENTSCHRIFT

(11) **EP 2 887 047 B1**
(45) Veröffentlichungstag und Bekanntmachung des Hinweises auf die Patenterteilung: **18.08.2021**
(21) Anmeldenummer: 13199187.9
(22) Anmeldetag: 20.12.2013
(51) Int. Cl.: G01N 3/04, G01N 3/24, G01N 33/44

(54) **Schubrahmen sowie Prüfeinrichtung und Verfahren zur Ermittlung wenigstens einer Eigenschaft eines Prüflings**
Push frame and test device and method for determining at least one property of a test piece
Cadre coulissant, dispositif de contrôle et procédé de détermination d'au moins une propriété d'un échantillon

(43) Veröffentlichungstag der Anmeldung: 24.06.2015
(73) Patentinhaber: Grasse Zur Ingenieurgesellschaft mbH, 14167 Berlin (DE)
(72) Erfinder: Grasse, Fabian, 14057 Berlin (DE); Zur, Malte, 12203 Berlin (DE)
(74) Vertreter: Schulz Junghans Patentanwälte PartGmbB

(56) Entgegenhaltungen:
- Dipl.-Ing Ricardo Basan: "Untersuchung der intralaminaren Schubeigenschaften von Faserverbundwerkstoffen mit Epoxidharzmatrix unter Berücksichtigung nichtlinearer Effekte", , 31. Dezember 2011 (2011-12-31), Seiten 1-152, XP055119685, ISBN: 978-3-98-142813-1 Gefunden im Internet: URL:http://www.bam.de/de/service/publikati onen/publikationen_medien/dissertationen/d iss_74_vt.pdf [gefunden am 2014-05-22]
- S Chaterjee ET AL: "AD-A273 561 Test Methods for Composies a Statut Report, Colume III. Shear Test Methods", , 30. Juni 1993 (1993-06-30), Seiten 1-181, XP055119880, Gefunden im Internet: URL:http://www.dtic.mil/dtic/tr/fulltext/u 2/a273561.pdf [gefunden am 2014-05-23]

## Beschreibung

Die vorliegende Erfindung betrifft einen Schubrahmen zur Ermittlung wenigstens einer Eigenschaft eines Prüflings bei Einwirkung einer Schubbelastung sowie eine Prüfeinrichtung zur Ermittlung wenigstens einer Eigenschaft eines Prüflings bei Einwirkung einer Schubbelastung. Außerdem betrifft die vorliegende Erfindung ein Verfahren zur Ermittlung wenigstens einer Eigenschaft eines Prüflings bei Einwirkung einer Schubbelastung.

Im Zuge der Entwicklung neuer Materialien, insbesondere Verbundmaterialien, ist es notwendig, die genauen mechanischen Eigenschaften derartiger Materialien zu bestimmen. In Abhängigkeit von der jeweiligen Ausgestaltung des Materials, wie z. B. der Anzahl und Ausrichtung unterschiedlicher Materiallagen in Verbundwerkstoffen, lassen sich die mechanischen Eigenschaften zum Teil nur ungenügend theoretisch ermitteln. Zu diesem Zweck werden die neuen Werkstoffe mechanischen Belastungsversuchen ausgesetzt, um z. B. bei Einwirkung einer bestimmten Kraft auf einen Werkstoff-Prüfling die entstehende Verformung zu messen und dadurch Rückschlüsse über die mechanischen Eigenschaften ziehen zu können. So werden z. B. Faserverbund-Platten Schubversuchen unterzogen, um entsprechende Schubkennwerte des Faserverbundwerkstoffes zu analysieren.

Es ist z. B. aus der DE 10 2010 006 163 A1 eine Vorrichtung für die mechanische Werkstoffprüfung zur Bestimmung der Schubkennwerte von Werkstoffen bekannt, bei der ein Prüfling in der Vorrichtung zur Bestimmung der Schubkennwerte geklemmt wird und anschließend einer Kraft zur Realisierung einer Schubbelastung ausgesetzt wird. Dabei wird der Prüfling an einem Schubrahmen angeordnet, der aus vier identischen Rahmenteilen und Drehgelenken besteht. Der Prüfling wird auf einer Seite des Schubrahmens mit die Rahmenteile umgreifenden Spannelementen fixiert.

Aus der DD 101 015 ist ein Verfahren und eine Vorrichtung zur Bestimmung der Schubfestigkeit eines Schubmoduls bekannt, bei dem ein im Wesentlichen viereckig und eben ausgeführter Prüfling, der an den Ecken mit Ausnehmungen versehen ist, in einer Einspannvorrichtung mit vier Drehpunkten und vier Klemmen aufgenommen wird. Durch Einleitung von entgegengesetzten Zugkräften in diagonal gegenüberliegenden Eckpunkten der Einspannvorrichtung wird der Prüfling auf Schub belastet. Dokumente von R. Basan: "Untersuchung der intralaminaren Schubeigenschaften von Faserverbundwerkstoffen mit Epoxidharzmatrix unter Berücksichtigung nichtlinearer Effekte", 2011-12-31, Seiten 1-15, ISBN: 978-3-98-142813-1 und S. Chaterjee: "Test Methods for Composies a Statut Report, Colume III. Shear Test Methods", 1993-06-30, Seiten 1-181, stellen den relevanten Stand der Technik dar.

Der vorliegenden Erfindung liegt die Aufgabe zugrunde, einen Schubrahmen, eine Prüfeinrichtung sowie ein Verfahren zur Ermittlung wenigstens einer Eigenschaft eines Prüflings bei Einwirkung einer Schubbelastung zur Verfügung zu stellen, mit denen in einfacher und kostengünstiger Weise ein Prüfling lediglich einer Schubbelastung aussetzbar ist, wobei die Bedienbarkeit der Vorrichtung und die Durchführung des Verfahren ergonomisch gestaltet ist.

Diese Aufgabe wird durch den erfindungsgemäßen Schubrahmen nach Anspruch 1, durch die erfindungsgemäße Prüfeinrichtung nach Anspruch 6 und durch das erfindungsgemäße Verfahren zur Ermittlung wenigstens einer Eigenschaft eines Prüflings bei Einwirkung einer Schubbelastung nach Anspruch 7 gelöst. Vorteilhafte Ausgestaltungen des erfindungsgemäßen Schubrahmens sind in den Unteransprüchen 2 - 5 angegeben. Vorteilhafte Ausgestaltungen des erfindungsgemäßen Verfahrens sind in den Unteransprüchen 8 und 9 angegeben.

Der Schubrahmen dient zur Ermittlung wenigstens einer Eigenschaft eines Prüflings bei Einwirkung einer Schubbelastung und umfasst zwei Rahmenhälften, zwischen denen ein Prüfling kraft- und/oder formschlüssig fixierbar ist, wobei sich die beiden Rahmenhälften im fixierenden Zustand im Wesentlichen in zwei zueinander parallel verlaufenden Ebenen erstrecken und jeweils vier Koppelelemente sowie vier Gelenke mit lediglich einem rotatorischen Freiheitsgrad aufweisen, mit denen die Koppelelemente miteinander gelenkig verbunden sind. Es ist erfindungsgemäß vorgesehen, dass ein jeweiliges Gelenk einer Rahmenhälfte des Schubrahmens auf der Linie seiner Drehachse von jedem Gelenk der gegenüberliegenden Rahmenhälfte separiert ist. Jede Rahmenhälfte weist vier Koppelelemente und nur vier Gelenke auf, die zusammen eine in sich geschlossene Koppelelement-Kette ausbilden. Die Gelenke sind dabei nur für eine Drehbewegung in einer Ebene ausgestaltet. Dabei können die Rahmenhälften derart ausgebildet sein, dass ein oder zwei Koppelelemente der jeweiligen Rahmenhälfte fest an einem Gestell bzw. einem Anschlusselement zur Einleitung von Kräften angeschlossen sind. Die beiden Rahmenhälften des Schubrahmens sind in zwei Ebenen angeordnet, die genau parallel zueinander verlaufen. Die Drehachse eines jeweiligen Gelenkes ist dabei die ideelle bzw. imaginäre Achse, um die herum eine Drehbewegung im Gelenk erfolgen kann. Das heißt, dass die Rahmenhälften im Bereich ihrer Gelenke mechanisch voneinander getrennt sind, so dass sich ein Prüfling auch zwischen den Gelenken erstrecken kann. Damit sind die Rahmenhälften jeweils mit mehreren Drehgelenken und dazwischen angeordneten Koppelelementen ausgebildet, wobei die gelenkige Verbindung zwischen den Koppelelementen derart realisiert ist, dass sich die Gelenknaben sowie die materiell vorhandenen Gelenkachsen nur in den jeweiligen Rahmenhälften erstrecken, in denen die jeweiligen Gelenke angeordnet sind, und nicht die Rahmenhälften miteinander verbinden. Die Gelenke sind vorzugsweise durch Gelenkbolzen in den entsprechenden Bohrungen in den Koppelelementen ausgebildet, so dass der Gelenkbolzen die Gelenkachse ausbildet und der den Gelenkbolzen umgebende Bereich des Koppelelementes die Gelenknabe ausbildet.

Der Vorteil des Schubrahmens liegt insbesondere darin, dass somit auch zwischen den Gelenken, die in einander gegenüberliegenden Rahmenhälften angeordnet sind, Material des Prüflings positionierbar ist, wie z. B. Rand- bzw. Eckbereiche des Prüflings. Somit können auch die Gelenke selbst bzw. Materialbereiche der die Gelenke aufnehmenden Koppelelemente zur Klemmung des Prüflings dienen, so dass auch Randzonen des Prüflings ungestört der Schubbelastung aussetzbar sind.

Vorzugsweise sind Gelenke der beiden Rahmenhälften paarweise einander gegenüberliegend in den beiden Ebenen der Rahmenhälfte angeordnet, wobei die Drehachsen der Drehgelenke des jeweiligen Gelenkpaares im Wesentlichen auf einer Achslinie angeordnet sind und die Gelenkachsen sowie die Gelenknaben der Gelenke des jeweiligen Gelenkpaares voneinander separiert sind. Das heißt, dass bei der paarweisen Anordnung der Gelenke diese ebenfalls nicht miteinander verbunden sind, sondern lediglich derart ausgerichtet sind, dass die ideellen Drehachsen der Gelenke des jeweiligen Gelenkpaares auf einer Linie angeordnet sind. Das heißt, dass noch in dieser Ausgestaltung weder die materiell vorhandenen Gelenkachsen, noch die Gelenknaben der Gelenke im Gelenkbereich miteinander verbunden sind. Üblicherweise wird dabei eine Gelenknabe eines Gelenkes durch einen Endbereich eines Koppelelementes ausgebildet, wobei die Gelenknabe in Längserstreckungsrichtung des Koppelelementes eine maximale Länge LGN aufweist die höchstens ein Drittel der Länge des Koppelelementes LKE beträgt. Auch bei dieser paarweisen Anordnung der Gelenke lässt sich ein Prüfling zwischen den Gelenken und somit im Verlauf der ideellen Drehachsen der Gelenke positionieren und zusammenpressen und demzufolge auch einer Schubbelastung aussetzen.

Um den Schubrahmen an eine Schubeinrichtung zur Aufbringung einer Zug- und/oder Druckkraft auf eine jeweilige Rahmenhälfte ankoppeln zu können weist der Schubrahmen erfindungsgemäß mindestens ein Anschlusselement auf, welches mittels wenigstens eines Anlenkbolzens mit einem Koppelement einer Rahmenhälfte verbunden ist, wobei das Anschlusselement dafür eingerichtet ist, an die Schubeinrichtung mechanisch angeschlossen zu werden und eine von der Schubeinrichtung bewirkte Zug- und/oder Druckkraft auf die das jeweilige Koppelement aufweisende Rahmenhälfte zu übertragen. Der Anlenkbolzen ist dabei derart lang ausgeführt, dass das daran angeschlossene Koppelelement und demzufolge auch die dieses Koppelelement umfassende Rahmenhälfte aufgrund einer Relativbewegung zwischen dem angeschlossenen Koppelement und dem Anlenkbolzen und/oder zwischen dem Anlenkbolzen und dem Anschlusselement translatorisch verschoben werden kann. Die Schubeinrichtung ist dabei eine Zug- und/oder Druckeinrichtung, die z. B. zwei im Wesentlichen koaxial zueinander angeordnete Kolben-Zylinder-Einheiten aufweist, mit denen bei Beaufschlagung mit einem geeigneten Fluid eine entsprechende Zug- oder Druckkraft aufbringbar ist. In alternativer Ausgestaltung ist die Schubeinrichtung eine elektrisch angetriebene Verfahr-Einrichtung mit einem Spindeltrieb zur Aufbringung der Zug- oder Druckkraft. Das zwischen einem Koppelelement und einer Kolben-Zylinder-Einheit bzw. Spindeltrieb angeordnete Anschlusselement ist vorzugsweise winkelförmig ausgestaltet. Zur Befestigung des Koppelelementes an einem solchen Anschlusselement dienen vorzugsweise jeweils mehrere Anlenkbolzen, die relativ lang ausgeführt sind und fest im Anschlusselement angeordnet sind, so dass die daran angeordnete Rahmenhälfte durch Gleitbewegung auf dem jeweiligen Schaft eines Anlenkbolzens translatorisch aus der Ebene der Position der Klemmung des Prüflings verschoben werden kann, um den Abstand zwischen den beiden Rahmenhälften zu vergrößern und in einfacher Weise der Austausch des Prüflings zwischen den Rahmenhälften zu ermöglichen. Die Anlenkbolzen sind dabei derart lang auszuführen, dass die beiden Rahmenhälften voneinander translatorisch zumindest auf einen Abstand von 30 mm entfernt werden können.

In weiterer vorteilhafter Ausgestaltung des erfindungsgemäßen Schubrahmens umfasst dieser wenigstens eine Presseinrichtung, mit der eine Rahmenhälfte mit mindestens einer Kraft in Richtung der anderen Rahmenhälfte beaufschlagbar ist, so dass ein Prüfling zwischen den Rahmenhälften durch den von den Rahmenhälften bewirkten Druck kraftschlüssig fixierbar ist. Vorzugsweise sollten mehrere Presseinrichtungen vorhanden sein, wie z. B. eine Presseinrichtung je Koppelelement. Die von der jeweiligen Presseinrichtung bewirkte Kraft wird auf das die Presseinrichtung tragende Koppelelement übertragen, so dass dieses Koppelelement eine Streckenlast bzw. Flächenlast zur Pressung des Prüflings auf diesen ausübt.

Vorzugsweise ist eine solche Presseinrichtung durch wenigstens eine Kolben-Zylinder-Einheit ausgebildet. In alternativen Ausführungsformen ist die Presseinrichtung elektrisch oder manuell mechanisch angetrieben.. In einer bevorzugten Ausgestaltung mit mehreren Kolben-Zylinder-Einheiten werden die Rahmenhälften somit mittels dieser Kolben-Zylinder-Einheiten, die als Hydraulik- oder auch als Pneumatikzylinder ausgestaltet sein können, zwecks Klemmung des Prüflings zwischen den Rahmenhälften zusammengezogen. Eine jeweilige Kolben-Zylinder-Einheit ist an einer Rahmenhälfte an der der jeweiligen anderen Rahmenhälfte gegenüberliegenden Seite angeordnet, wobei sich ein Hohlkolben der Kolben-Zylinder-Einheit durch die sie tragende Rahmenhälfte und durch die gegenüberliegende Rahmenhälfte hindurchgeführt ist und an der dem Zylinder der Kolben-Zylinder-Einheit abgewandten Seite dieser Rahmenhälfte an dieser abstützt, wie z. B. mittels einer geeigneten Verschraubung. Bei Betätigung der jeweiligen Kolben-Zylinder-Einheit lässt sich der Hohlkolben herausfahren, so dass er sich einerseits an dem Koppelelement der Rahmenhälfte abstützt, an dem die Kolben-Zylinder-Einheit befestigt ist, und andererseits eine Zugkraft über die Verschraubung auf die andere Rahmenhälfte überträgt, so dass die beiden Rahmenhälften mit einem entsprechenden Kräftepaar beaufschlagt werden, welches die Klemmung eines Prüflings zwischen den Rahmenhälften ermöglicht. Die jeweiligen Kolben-Zylinder-Einheiten sind bevorzugt mittig an den Koppelelementen angeordnet und weisen somit einen maximalen Abstand zu den jeweils benachbarten Gelenken auf.

Wie bereits erwähnt ist der Kolben einer jeweiligen Kolben-Zylinder-Einheit vorzugsweise als Hohlkolben ausgeführt, so dass in diesem Hohlkolben ein Positionierungswerkzeug führbar ist, welches passgenau im Hohlkolben der Kolben-Zylinder-Einheit aufnehmbar ist und passgenau in eine Positionierungsbohrung des Prüflings ein- oder durchsteckbar ist, um den Prüfling in einer exakten Position im Bezug zu den Rahmenhälften zu positionieren. Die Passung zwischen dem Positionierungswerkzeug und der Bohrung im Prüfling ist vorzugsweise H7-h6 nach DIN 7154 T1. Im Hohlkolben ist das Positionierungswerkzeug im Wesentlichen spielfrei geführt. Zur Positionierung des Prüflings im Schubrahmen kann alternativ oder auch zusätzlich vorgesehen sein, dass der Prüfling mittels Anlage einer oder mehrerer seiner Außenkanten an einem bzw. mehreren Anlageelementen des Schubrahmens positionierbar ist.

In ähnlicher Weise, wie im Hohlkolben einer Kolben-Zylinder-Einheit ein Positionierungswerkzeug aufnehmbar ist, um den Prüfling zu positionieren, ist zum Zweck der beschriebenen Krafteinwirkung auf die Rahmenhälften zwecks Pressung des Prüflings in einem jeweiligen Hohlkolben ein Zugbolzen angeordnet, der durch den Prüfling hindurchführt und sich auf der dem Zylinder der Kolben-Zylinder-Einheit gegenüberliegenden Rahmenhälfte abstützt, wie z. B. durch eine Mutter, die auf den Zugbolzen aufgeschraubt ist. Bei der beschriebenen Betätigung der Kolben-Zylinder-Einheit und Ausfahren des Hohlkolbens zieht dieser somit den Zugbolzen, so dass die auf den Zugbolzen aufgeschraubte Mutter gegen die an ihr anliegende Rahmenhälfte drückt.

Zur Einstellung einer exakten Ausrichtung der Koppelelemente der Rahmenhälften zwecks Erleichterung der Positionierung eines Prüflings Schubrahmen weist dieser in der Nähe wenigstens eines Gelenkes im Überlappungsbereich von zwei Koppelelementen in diesen beiden Koppelelementen jeweils eine Prüfbohrung mit gleichem Durchmesser auf, die einander überdecken, wenn die Koppelelemente in einer Position zueinander angeordnet sind, in der ein Prüfling im unverformten Zustand in dem Schubrahmen aufnehmbar ist, so dass auch Öffnungen bzw. Bohrungen im Prüfling zur Hindurchführung des Positionierungswerkzeuges bzw. eines Zugbolzens jeweils im Wesentlichen koaxial zu jeweils einer Längsachse eines Hohlkolbens einer Kolben-Zylinder-Einheit angeordnet sind. Durch Einführung eines Stiftes bzw. Bolzens mit einer geeigneten Passung in ein solches Prüfbohrungspaar lässt sich feststellen, ob sich die damit versehenen Koppelelemente in der gewünschten Ausgangsposition zur Aufnahme des Prüflings befinden.

Vorzugsweise ist an wenigstens einer Rahmenhälfte an jedem Koppelelement ein Sockelelement zur Aufnahme einer mittels Senkschrauben mit dem jeweiligen Sockelelement verschraubten Kolben-Zylinder-Einheit bzw. zur Anlage eines den Zugbolzen abstützenden Elementes, wie z. B. der Mutter auf dem Zugbolzen, angeordnet. Ein solches Sockelelement kann einen verlängerten Bereich in Form einer Lasche umfassen, der in Richtung der Längsachse eines Gelenkbolzens an diesem anliegt bzw. in dieser Richtung gegen den Gelenkbolzen drückt, um eine axiale Verschiebung des Bolzens zu verhindern.

Zur Lösung der Aufgabe wird weiterhin eine Prüfeinrichtung zur Ermittlung wenigstens einer Eigenschaft eines Prüflings bei Einwirkung einer Schubbelastung zur Verfügung gestellt, welche einen erfindungsgemäßen Schubrahmen sowie einen Prüfling umfasst, der sich in einer Ebene erstreckt und im Wesentlichen zwölfeckig ausgebildet ist, wobei er vier äußere Begrenzungskanten aufweist, die jeweils zu ihren jeweiligen benachbarten äußeren Begrenzungskanten im Wesentlichen senkrecht verlaufen und durch winklige Ausschnitte getrennt sind, wobei der Scheitelpunkt eines jeweiligen winkligen Ausschnittes auf einer Diagonalen liegt, die diametral gegenüberliegende Schnittpunkte von an benachbarten äußeren Begrenzungskanten angelegten Gerade verbindet, und die Drehachsen der paarweise angeordneten Gelenke der Rahmenhälften im Wesentlichen durch die Scheitelpunkte der winkligen Ausschnitte verlaufen. Vorzugsweise verlaufen die Gelenkachsen genau durch die Scheitelpunkte der winkligen Ausschnitte des Prüflings. Die winkligen Ausschnitte trennen die Begrenzungskanten, deren Verlängerungsgeraden den Schnittpunkt bilden, durch den die Diagonale führt, auf dem der Scheitelpunkt liegt. Der winklige Ausschnitt ist vorzugsweise spiegelsymmetrisch zu einer jeweiligen Diagonale ausgeführt. Dadurch wird ein Prüfling zur Verfügung gestellt, der eine ähnliche Form wie die eines Malteserkreuzes aufweist. Jedem Gelenkpaar ist vorzugsweise ein Scheitelpunkt eines winkligen Ausschnittes des Prüflings zugeordnet, wobei die jeweilige Gelenkachse genau durch den jeweils tiefsten Punkt der "Kerbe" zwischen den Malteserkreuz-Blättern verläuft.

Zur Lösung der Aufgabe wird weiterhin ein Verfahren zur Ermittlung wenigstens einer Eigenschaft eines Prüflings bei Einwirkung einer Schubbelastung zur Verfügung gestellt, bei dem ein Prüfling in einen erfindungsgemäßen Schubrahmen eingespannt wird und mittels Beaufschlagung des Schubrahmens mit einem Kräftepaar eine Schubspannung in den Prüfling eingebracht wird. Das erfindungsgemäße Verfahren ist demzufolge mittels der erfindungsgemäßen Prüfeinrichtung durchzuführen. Zwecks Positionierung ist dabei vorzugsweise der Prüfling mittels zwei in Hohlkolben von Kolben-Zylinder-Einheiten passgenau aufgenommenen Positionierungswerkzeugen in Bezug zu den Rahmenhälften positionieren, indem ein Abschnitt des jeweiligen Positionierungswerkzeuges passgenau in eine Positionierungsbohrung des Prüflings gesteckt wird und danach die Rahmenhälften zusammengepresst werden, so dass der Prüfling kraftschlüssig fixiert ist.

Zwecks Herausnahme und Einlegens des Prüflings in den Raum zwischen den Rahmenhälften lässt sich in ergonomisch vorteilhafter Weise eine translatorische Relativbewegung zwischen einem an einem Anlenkbolzen angeschlossenen Koppelelement und dem Anlenkbolzen und/oder zwischen dem Anlenkbolzen und einem an den Anlenkbolzen angeschlossenen Anschlusselement ausführen, um somit den Abstand zwischen den Rahmenhälften zu verändern. Durch die mechanische Verbindung des Anschlusselementes mit dem Anlenkbolzen und der mechanischen Verbindungen des Anlenkbolzens mit einem Koppelelement sowie durch die relativ große Länge eines jeweiligen Anlenkbolzens kann dieser als Linearführung dienen, um den Abstand zwischen dem Anschlusselement und dem Koppelelement zu variieren, ohne dass dabei die Gewichtskraft der verschobenen Rahmenhälfte von einer Bedienperson aufgenommen werden muss. Dabei sollte der Anlenkbolzen entweder fest an dem Anschlusselement und verschiebbar in dem Koppelelement angeordnet sein, oder umgekehrt verschiebbar im Anschlusselement und fest am Koppelelement angeordnet sein. Bei einer derartigen, vom Anlenkbolzen gestützten Bewegung einer Rahmenhälfte muss diese Rahmenhälfte demzufolge nicht komplett demontiert bzw. manuell von der anderen Rahmenhälfte entfernt und getragen werden, um einen Prüfling zwischen die Rahmenhälften einzulegen bzw. von dort zu entfernen. Gegenüber herkömmlichen Schubrahmen bzw. mit den Schubrahmen realisierten Verfahren zur Ermittlung wenigstens einer Eigenschaft eines Prüflings bei Einwirkung einer Schubbelastung lässt sich dadurch ca. 30 % der Zeit beim Prüflingswechsel einsparen.

Die Erfindung wird im Folgenden anhand der in den beiliegenden Zeichnungen dargestellten Ausführungsbeispiele erläutert.

Es zeigt:
- Fig. 1: einen Schubrahmen, der nicht Teil der Erfindung ist, in perspektivischer Ansicht,
- Fig. 2: die zweite Rahmenhälfte des Schubrahmens in perspektivischer Ansicht,
- Fig. 3: die zweite Rahmenhälfte des Schubrahmens in Draufsicht, und
- Fig. 4: einen Prüfling in Draufsicht.

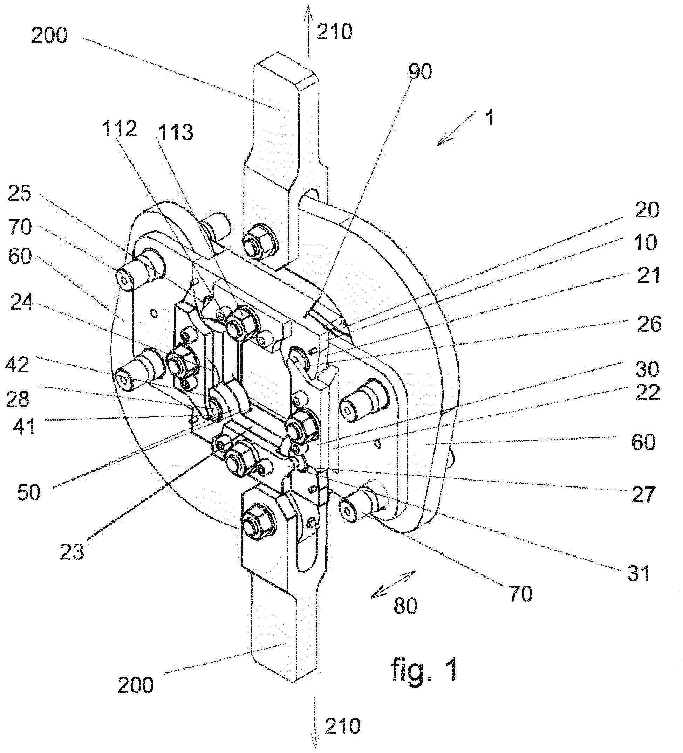

In Figur 1 ist der Schubrahmen 1 perspektivisch dargestellt, wobei hier die erste Rahmenhälfte 10 dem Betrachter zugewandt ist. Die erste Rahmenhälfte 10 umfasst ein erstes Koppelelement 21, ein zweites Koppelelement 22, ein drittes Koppelelement 23 und ein viertes Koppelelement 24. Zwischen dem ersten Koppelelement 21 und dem vierten Koppelelement 24 ist ein erstes Gelenk 25 angeordnet. Zwischen dem ersten Koppelelement 21 und dem zweiten Koppelelement 22 ist ein zweites Gelenk 26 angeordnet. Zwischen dem zweiten Koppelelement 22 und dem dritten Koppelelement 23 ist ein drittes Gelenk 27 angeordnet und zwischen dem dritten Koppelelement 23 und dem vierten Koppelelement 24 ist ein viertes Gelenk 28 angeordnet. Die Koppelelemente 21 - 24 bilden zusammen mit den Gelenken 25 - 28 eine in sich geschlossene Koppelelement-Kette aus. Das zweite Koppelelement 22 und das vierte Koppelelement 24 sind über Anlenkbolzen 70 mit jeweils einem Anschlusselement 60 verbunden, welches, wie in Fig. 1 beispielhaft dargestellt, im Wesentlichen als Winkel ausgeführt ist. An die Anschlusselemente 60 sind Zug-Elemente 200 angeschlossen, in die von einer hier nicht dargestellten Zugeinrichtung Zugkräfte 210 einleitbar sind. Bei Einleitung der Zugkräfte 210 werden diese über die Anschlusselemente 60 in das zweite Koppelelement 22 und das vierte Koppelelement 24 derart übertragen, dass die jeweiligen Rahmenhälften 10, 20 sich als Parallelogramm verformen und somit eine Schubbelastung in einen zwischen den Rahmenhälften 10, 20 eingespannten Prüfling einbringen. Der Prüfling selbst ist zwecks besserer Übersichtlichkeit nicht in Figur 1 dargestellt

In Fig. 1 ist die zweite Rahmenhälfte 20 nur ansatzweise zu erkennen, da sie hinter der Ebene, in der die Anschlusselemente 60 angeordnet sind, positioniert ist. Die Gelenke 25 - 28 einer jeweiligen Rahmenhälfte 10, 20 sind dabei paarweise in den beiden Ebenen, in denen sich die beiden Rahmenhälften 10, 20 erstrecken angeordnet. Das heißt, dass gegenüber dem ersten Gelenk 25 der ersten Rahmenhälfte 10 das erste Gelenk 25 der zweiten Rahmenhälfte 20 angeordnet ist. Die Gelenke sind dabei durch jeweils eine materiell in Form eines Gelenkbolzens realisierte Gelenkachse 41 und durch den diese Gelenkachse 41 umgebenden Bereich eines jeweiligen Koppelelementes 21 - 24, der die Gelenknabe 42 ausbildet, realisiert.

Die imaginären Drehachsen 40 der beiden Gelenke eines Gelenkpaares 50 verlaufen dabei im Wesentlichen und vorzugsweise genau auf einer Linie. Dabei sind sowohl die Gelenkachsen 41 als auch die Gelenknaben 42 der Gelenkpaare 50 voneinander separiert, so dass zwischen den Gelenken eines Gelenkpaares 50 ein Abschnitt eines Prüflings angeordnet werden kann.

Wie bereits erwähnt sind das zweite Koppelelement 22 und das vierte Koppelement 24 mit jeweils einem Anschlusselement 60 mittels Anlenkbolzen 70 verbunden. Diese Anlenkbolzen 70 sind erfindungsgemäß derart lang ausgeführt, dass sie als Linearführungen dienen können, so dass wenigstens eine der Rahmenhälfte 10, 20 in einer Richtung 80 in einer translatorischen Bewegung verschoben werden kann, so dass sich der Abstand 90 zwischen den Rahmenhälften 10, 20 vergrößern lässt. Dadurch wird der Raum zwischen den Rahmenhälften 10, 20 manuell leicht zugängig, ohne dass das Gewicht einer oder sogar beider Rahmenhälften 10, 20 manuell oder von einer Hilfsvorrichtung aufgenommen werden muss.

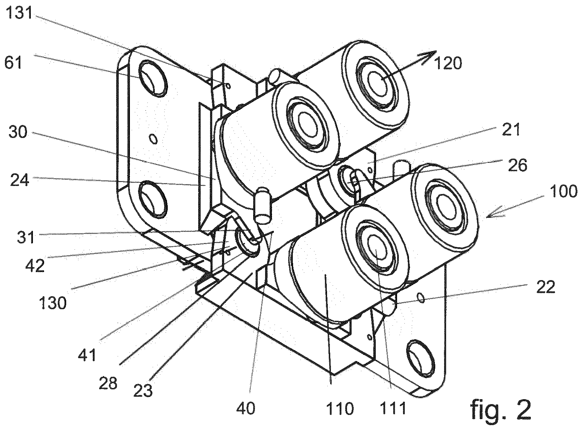

Fig. 2 zeigt die zweite Rahmenhälfte 10, die in Fig. 1 nur andeutungsweise ersichtlich ist. An der zweiten Rahmenhälfte 20 sind an den vier Koppelelementen 21 - 24 jeweils mittig Presseinrichtungen 100 angeordnet. Diese Presseinrichtungen 100 umfassen Kolben-Zylinder-Einheiten 110, die mit einem Hohlkolben 111 ausgestattet sind. Bei Betätigung der Kolben-Zylinder-Einheit 110 bewirkt diese eine Kraft 120 in der dargestellten Richtung. Ein in einem jeweiligen Hohlkolben 111 und hier aus Gründen der Übersichtlichkeit nicht dargestellter Zugbolzen wird somit von der zweiten Rahmenhälfte 20 weggeschoben.

Wie aus Fig. 1 ersichtlich ist, führt ein jeweiliger Zugbolzen 112 durch beide Rahmenhälften 10, 20 sowie auch durch den Prüfling hindurch und ist an der Außenseite der ersten Rahmenhälfte 10 mit einer Mutter 113 verschraubt. Mit der beschriebenen Krafteinwirkung von der Kolben-Zylinder-Einheit 110 auf den Zugbolzen 112 wird dieser somit auch von der ersten Rahmenhälfte 10 weggezogen. Durch die Abstützung der Mutter 113 an der ersten Rahmenhälfte 10 wird somit von der Kolben-Zylinder-Einheit ein Kräftepaar auf die beiden Rahmenhälften 10, 20 realisiert, welches zu einer Pressung des zwischen den Rahmenhälften 10, 20 angeordneten Prüflings führt.

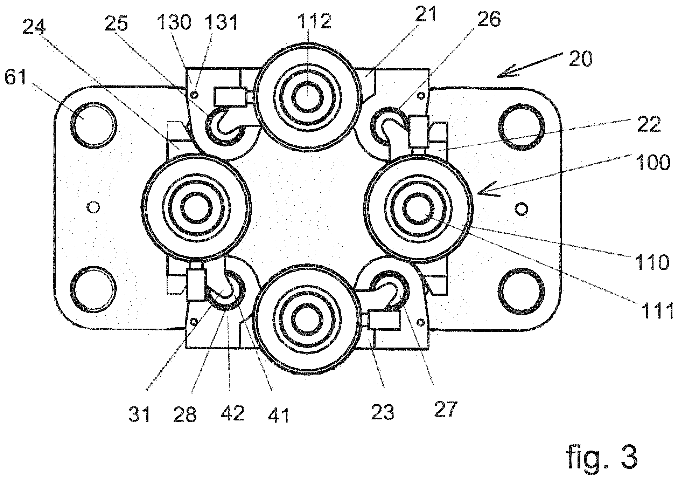

In den Figuren 2 und 3 sind zudem die Anschlussbohrungen 61 im zweiten Koppelelement 22 und im vierten Koppelelement 24 erkennbar, in denen jeweils ein Anlenkbolzen 70 führbar ist. Außerdem sind Sockelelemente 30 an den Koppelelementen 21 -24 ersichtlich, auf denen sich die Kolben-Zylinder-Einheiten 110 abstützen. Diese Sockelelemente 30 haben jeweils eine einseitige Verlängerung in Form einer Lasche 31, die an den Gelenkachsen 41 in Richtung derer Drehachsen 40 anliegen, um bei Belastung des Schubrahmens eine axiale Verschiebung der Gelenkachsen 41 zu verhindern.

Weiterhin ist ersichtlich, dass im Überlappungsbereich 130 zwischen zwei Koppelelementen Prüfbohrungen 131 angeordnet sind, die bei genauer rechtwinkliger Anordnung z. B. des ersten Koppelelementes 21 im Bezug zum vierten Koppelelement 24 exakt übereinander liegen, so dass ein passender Stift mit Übergangspassung in diesen Prüfbohrungen 131 eingeführt werden kann um bei vollständiger Aufnahme in diese Prüfbohrungen 131 die Feststellung der exakten Positionierung der Koppelelemente zu signalisieren.

Das Detail dieser Prüfbohrung 131 ist ebenfalls in Fig. 3 erkennbar.

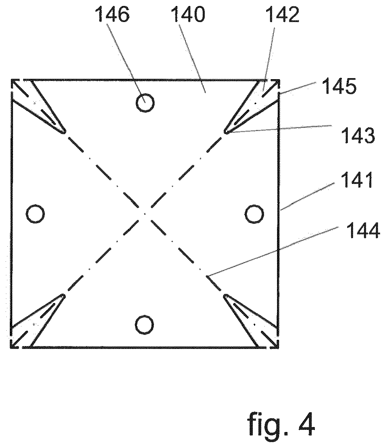

In Fig. 4 ist ein Prüfling 140 dargestellt, der Ähnlichkeit mit einem typischen Malteserkreuz aufweist. Der Prüfling 140 umfasst 4 äußere Begrenzungskanten 141, die im Wesentlichen rechtwinklig bzw. parallel zueinander verlaufen. Zwischen benachbarten Begrenzungskanten 141 sind winklige Ausschnitte 142 angeordnet. Die Scheitelpunkte dieser winkligen Ausschnitte 142 liegen auf Diagonalen 144, die Eckpunkte von an benachbarten äußeren Begrenzungskanten angelegten Geraden 145 miteinander verbinden. Im Wesentlichen symmetrisch sind Positionierungsbohrungen 146 im Prüfling eingebracht.

Der Prüfling 140 wird derart in den Schubrahmen 1 eingelegt, dass die Drehachsen 40 der jeweiligen Gelenkpaare 50 durch die Scheitelpunkte 143 der winkligen Ausschnitte 142 im Prüfling 140 verlaufen.

Zwecks Positionierung des Prüflings 140 zwischen den Rahmenhälften 10, 20 vor Durchführung des Schubversuches kann anstelle eines Zugbolzens 112 in einer Kolben-Zylinder-Einheit 110 ein Positionierungswerkzeug eingesetzt werden, welches im Wesentlichen passgenau im Hohlkolben der Kolben-Zylinder-Einheit 110 aufgenommen ist. Dieses Positionierungswerkzeug umfasst einen endseitigen Abschnitt, der passgenau in einer Positionierungsbohrung 146 des Prüflings 140 aufnehmbar ist. Bei exakter Ausrichtung der Koppelelemente 21 --24 zueinander, wie es z. B. mittels der Prüfbohrungen 131 feststellbar ist, lassen sich durch die Hohlkolben der Kolben-Zylinder-Einheiten 110 die genannten Positionierungswerkzeuge hindurchführen und auf diese der Prüfling 140 mit seinen Positionierungsbohrungen 146 stecken. Nach Verringerung des Abstandes der beiden Rahmenhälften 10, 20 zueinander aufgrund der translatorischen Bewegung entlang der Richtung 80 auf den Anlenkbolzen 70 als Linearführungen lässt sich der Prüfling 140 mittels der Rahmenhälften 10, 20 einem leichten Anpassdruck aussetzen, der groß genug ist, um den Prüfling zwischen den Rahmenhälften 10, 20 zu fixieren. Danach lassen sich die Positionierungswerkzeuge aus den Kolben-Zylinder-Einheiten 110 entfernen und die beschriebenen Zugbolzen 112 in die Hohlkolben 111 der Kolben-Zylinder-Einheiten 110 einsetzen und mit der Mutter an der gegenüberliegenden Rahmenhälfte 10 verschrauben. Durch Betätigung der Kolben-Zylinder-Einheiten 110 lassen sich die Rahmenhälften 10, 20 zusammendrücken, so dass der Prüfling positionsgesichert durch Einleitung der Zugkräfte 210 der Schubbelastung ausgesetzt werden kann.

### Bezugszeichenliste

| | |
|---|---|
| Schubrahmen | 1 |
| Erste Rahmenhälfte | 10 |
| Zweite Rahmenhälfte | 20 |
| Erstes Koppelelement | 21 |
| Zweites Koppelelement | 22 |
| Drittes Koppelelement | 23 |
| Viertes Koppelelement | 24 |
| Erstes Gelenk | 25 |
| Zweites Gelenk | 26 |
| Drittes Gelenk | 27 |
| Viertes Gelenk | 28 |
| Sockelelement | 30 |
| Lasche | 31 |
| Drehachse | 40 |
| Gelenkachse | 41 |
| Gelenknabe | 42 |
| Gelenkpaar | 50 |
| Anschlusselement | 60 |
| Anschlussbohrung | 61 |
| Anlenkbolzen | 70 |
| Richtung der translatorischen Bewegung | 80 |
| Abstand | 90 |
| Presseinrichtung | 100 |
| Kolben-Zylinder-Einheit | 110 |
| Hohlkolben | 111 |
| Zugbolzen | 112 |
| Mutter | 113 |
| Kraft | 120 |
| Überlappungsbereich | 130 |
| Prüfbohrung | 131 |
| Prüfling | 140 |
| äußere Begrenzungskante | 141 |
| winkliger Ausschnitt | 142 |
| Scheitelpunkt | 143 |
| Diagonale | 144 |
| an äußerer Begrenzungskanten angelegte Gerade | 145 |
| Positionierungsbohrung | 146 |
| Zug-Element | 200 |
| Zugkraft | 210 |

## Patentansprüche

1. Schubrahmen (1) zur Ermittlung wenigstens einer Eigenschaft eines Prüflings (140) bei Einwirkung einer Schubbelastung, umfassend zwei Rahmenhälften (10,20), zwischen denen ein Prüfling (140) kraft- und/ oder formschlüssig fixierbar ist, wobei sich die beiden Rahmenhälften (10,20) im fixierenden Zustand im Wesentlichen in zwei zueinander parallel verlaufenden Ebenen erstrecken und jeweils vier Koppelelemente (21,22,23,24) sowie vier Gelenke (25,26,27,28) mit lediglich einem rotatorischen Freiheitsgrad aufweisen, mit denen die Koppelelemente (21,22,23,24) miteinander gelenkig verbunden sind,
wobei
ein jeweiliges Gelenk (25,26,27,28) einer Rahmenhälfte (10,20) auf der Linie seiner Drehachse (40) von jedem Gelenk (25,26,27,28) der gegenüberliegenden Rahmenhälfte (10,20) separiert ist, wobei der Schubrahmen (1) wenigstens ein Anschlusselement (60) aufweist, welches mittels mindestens eines Anlenkbolzens (70) mit einem Koppelelement (22,24) verbunden ist, wobei das Anschlusselement (70) dafür eingerichtet ist, an eine Schubeinrichtung mechanisch angeschlossen zu werden und eine Zug-und/ oder eine Druckkraft (210) von der Schubeinrichtung auf die das jeweilige Koppelelement (22,24) aufweisende Rahmenhälfte (10,20) zu übertragen, und **dadurch gekennzeichnet, dass** der Anlenkbolzen (70) derart lang ausgeführt ist, dass das daran angeschlossene Koppelement (22,24) und demzufolge auch die dieses Koppelelement (22,24) umfassende Rahmenhälfte (10,20) auf Grund einer Relativbewegung zwischen dem angeschlossenen Koppelelement (22,24) und dem Anlenkbolzen (70) und/ oder zwischen dem Anlenkbolzen (70) und dem Anschlusselement (60) translatorisch verschoben werden kann.

2. Schubrahmen nach Anspruch 1,
**dadurch gekennzeichnet,**
**dass** Gelenke (25,26,27,28) der beiden Rahmenhälften (10,20) paarweise einander gegenüberliegend angeordnet sind, wobei die Drehachsen (40) der Gelenke (25,26,27,28) des jeweiligen Gelenkpaares (50) im Wesentlichen auf einer Achslinie angeordnet sind und die Gelenkachsen (41) und die Gelenknaben (42) der Gelenke (25,26,27,28) des jeweiligen Gelenkpaares (50) voneinander separiert sind.

3. Schubrahmen nach einem der vorhergehenden Ansprüche,
**dadurch gekennzeichnet,**
**dass** er wenigstens eine Presseinrichtung (100) umfasst, mit der eine Rahmenhälfte (10,20) mit mindestens einer Kraft (120) in Richtung der anderen Rahmenhälfte (10,20) beaufschlagbar ist, so dass ein Prüfling (140) zwischen den Rahmenhälften (10,20) durch den von den Rahmenhälften (10,20) bewirkten Druck kraftschlüssig fixierbar ist.

4. Schubrahmen nach Anspruch 3,
**dadurch gekennzeichnet,**
**dass** die Presseinrichtung (100) durch wenigstens eine Kolben-Zylinder-Einheit (110) ausgebildet ist.

5. Schubrahmen nach Anspruch 4,
**dadurch gekennzeichnet,**
**dass** der Kolben der Kolben-Zylinder-Einheit (110) als Hohlkolben (111) ausgeführt ist und dass der Schubrahmen (1) ein Positionierungswerkzeug umfasst, welches passgenau im Hohlkolben (111) der Kolben-Zylinder-Einheit (110) aufnehmbar ist und passgenau in eine Positionierungsbohrung (146) des Prüflings ein- oder durchsteckbar ist, um den Prüfling (140) in einer exakten Position in Bezug zu den Rahmenhälften (10,20) zu positionieren.

6. Prüfeinrichtung zur Ermittlung wenigstens einer Eigenschaft eines Prüflings (140) bei Einwirkung einer Schubbelastung, umfassend einen Schubrahmen (1) gemäß einem der Ansprüche 2 bis 5 sowie einen Prüfling (140), der sich in einer Ebene erstreckend im Wesentlichen zwölfeckig ausgebildet ist, wobei er vier äußere Begrenzungskanten (141) aufweist, die zu ihren jeweiligen benachbarten äußeren Begrenzungskanten (141) im Wesentlichen senkrecht verlaufen und durch winklige Ausschnitte (142) getrennt sind, wobei der Scheitelpunkt (143) eines jeweiligen winkligen Ausschnittes (142) auf einer Diagonalen (144) liegt, die diametral gegenüberliegende Schnittpunkte von an benachbarten äußeren Begrenzungskanten (141) angelegten Geraden (145) verbindet, und die Drehachsen (40) der paarweise angeordneten Gelenke (25,26,27,28) der Rahmenhälften (10,20) im Wesentlichen durch die Scheitelpunkte (143) der winkligen Ausschnitte (142) verlaufen.

7. Verfahren zur Ermittlung wenigstens einer Eigenschaft eines Prüflings (140) bei Einwirkung einer Schubbelastung, bei dem ein Prüfling (140) in einen Schubrahmen (1) gemäß einem der Ansprüche 1 bis 5 eingespannt wird und mittels Beaufschlagung des Schubrahmens 1 mit einem Kräftepaar eine Schubspannung in den Prüfling (140) eingebracht wird.

8. Verfahren zur Ermittlung wenigstens einer Eigenschaft eines Prüflings bei Einwirkung einer Schubbelastung nach Anspruch 7,
**dadurch gekennzeichnet,**
**dass** der Prüfling (140) mittels wenigstens zwei in als Hohlkolben (111) ausgestalteten Kolben von Kolben-Zylinder-Einheiten (110) passgenau aufgenommenen Positionierungswerkzeugen in Bezug zu den Rahmenhälften (10,20) positioniert wird, indem ein Abschnitt des jeweiligen Positionierungswerkzeuges passgenau in eine Positionierungsbohrung (146) des Prüflings (140) gesteckt wird und danach die Rahmenhälften (10,20) zusammengepresst werden, so dass der Prüfling (140) kraftschlüssig fixiert ist.

9. Verfahren zur Ermittlung wenigstens einer Eigenschaft eines Prüflings (140) bei Einwirkung einer Schubbelastung nach einem der Ansprüche 7 und 8,
**dadurch gekennzeichnet,**
**dass** zwecks Herausnahme oder Einlegen des Prüflings (140) in den Raum zwischen den Rahmenhälften (10,20) eine translatorische Relativbewegung zwischen einem an ein Anlenkbolzen (70) angeschlossenem Koppelelement (22,24) und dem Anlenkbolzen (70) und/ oder zwischen dem Anlenkbolzen (70) und einem an den Anlenkbolzen (70) angeschlossenem Anschlusselement (60) ausgeführt wird, um den Abstand zwischen den Rahmenhälften (10,20) zu verändern.

## Claims

1. A push frame (1) for determining at least one property of a test piece (140) upon impact of a thrust load, comprising two frame halves (10, 20) between which a test piece (140) is non-positively and/or positively fixable, wherein, when in the fixing state, the two frame halves (10, 20) extend substantially in two planes running parallel to each other and each have four coupling elements (21, 22, 23, 24) as well as four joints (25, 26, 27, 28) with only one rotational degree of freedom, with which joints the coupling elements (21, 22, 23, 24) are articulated to each other,
wherein
a respective joint (25, 26, 27, 28) of a frame half (10, 20) is separated from each joint (25, 26, 27, 28) of the opposing frame half (10, 20) on the line of its rotational axis (40),
wherein the push frame (1) has at least one linking element (60) which is connected to a coupling element (22, 24) by means of at least one linkage bolt (70), wherein the linking element (70) is adapted to be mechanically linked to a push device and to transfer a tensile and/or a compressive force (210) of the push device to the frame half (10, 20) including the respective coupling element (22, 24), and **characterized in that** the linkage bolt (70) is designed with a length such that the coupling element (22, 24) linked thereto and thus also the frame half (10, 20) comprising said coupling element (22, 24) can be translationally displaced due to a relative movement between the linked coupling element (22, 24) and the linkage bolt (70) and/or between the linkage bolt (70) and the linking element (60).

2. The push frame according to Claim 1,
**characterized in that**
joints (25, 26, 27, 28) of the two frame halves (10, 20) are arranged opposite each other in pairs, wherein the rotational axes (40) of the joints (25, 26, 27, 28) of the respective joint pair (50) are substantially arranged on one axis line and the joint axes (41) and the joint hubs (42) of the joints (25, 26, 27, 28) of the respective joint pair (50) are separated from each other.

3. The push frame according to any one of the preceding claims,
**characterized in that**
it comprises at least one pressing device (100) with which at least one force (120) is exertable on the one frame half (10, 20) in the direction of the other frame half (10, 20) such that a test piece (140) is non-positively fixable between the frame halves (10, 20) by the pressure caused by the frame halves (10, 20).

4. The push frame according to Claim 3,
**characterized in that**
the pressing device (100) is formed by at least one piston-cylinder unit (110).

5. The push frame according to Claim 4,
**characterized in that**
the piston of the piston-cylinder unit (110) is designed as a hollow piston (111) and **in that** the push frame (1) comprises a positioning tool which is precisely accommodatable in the hollow piston (111) of the piston-cylinder unit (110) and is precisely insertable or pluggable into a positioning bore (146) of the test piece in order to position the test piece (140) in an exact position relative to the frame halves (10, 20).

6. A test device for determining at least one property of a test piece (140) upon impact of a thrust load, comprising a push frame (1) according to any one of Claims 2 to 5 as well as a test piece (140) extending in one plane and being substantially dodecagonally formed, wherein it has four outer delimiting edges (141) which run substantially perpendicularly to their respective neighboring outer delimiting edges (141) and are separated by angled cutouts (142), wherein the vertex (143) of a respective angled cutout (142) lies on a diagonal (144) which connects diametrically opposing intersection points of straight lines (145) abutting neighboring outer delimiting edges (141), and the rotational axes (40) of the joints (25, 26, 27, 28) of the frame halves (10, 20) arranged in pairs substantially run through the vertices (143) of the angled cutouts (142).

7. A method for determining at least one property of a test piece (140) upon impact of a thrust load, wherein a test piece (140) is clamped in a push frame (1) according to any one of Claims 1 to 5 and a shear stress is introduced into the test piece (140) by means of exerting a pair of forces on the push frame 1.

8. The method for determining at least one property of a test piece upon impact of a thrust load according to Claim 7,
**characterized in that**
the test piece (140) is positioned relative to the frame halves (10, 20) by means of at least two positioning tools precisely accommodated in pistons of piston-cylinder units (110) configured as hollow pistons (111) by precisely inserting a portion of the respective positioning tool into a positioning bore (146) of the test piece (140) and then pressing the frame halves (10, 20) together so that the test piece (140) is non-positively fixed.

9. The method for determining at least one property of a test piece (140) upon impact of a thrust load according to any one of Claims 7 and 8,
**characterized in that**,
for purposes of removing the test piece (140) from the space between the frame halves (10, 20) or placing it therein, a translational relative movement is carried out between a coupling element (22, 24) linked to a linkage bolt (70) and the linkage bolt (70) and/or between the linkage bolt (70) and a linking element (60) linked to the linkage bolt (70) in order to change the distance between the frame halves (10, 20).

## Revendications

1. Cadre coulissant (1) servant à déterminer au moins une propriété d'un échantillon (140) lors de l'action d'une contrainte de poussée, comprenant deux moitiés de cadre (10, 20), entre lesquelles un échantillon (140) peut être fixé par adhérence et/ou complémentarité de forme, dans lequel les deux moitiés de cadre (10, 20) s'étendent pratiquement dans deux plans parallèles l'un à l'autre à l'état fixé et présentent respectivement quatre éléments de couplage (21, 22, 23, 24) ainsi que quatre articulations (25, 26, 27, 28) avec seulement un degré de liberté en rotation, avec lesquels les éléments de couplage (21, 22, 23, 24) sont reliés les uns aux autres de façon articulée,
dans lequel
une articulation respective (25, 26, 27, 28) d'une moitié de cadre (10, 20) est séparée de chaque articulation (25, 26, 27, 28) de la moitié de cadre opposée (10, 20) sur la ligne de son axe de rotation (40),
dans lequel le cadre coulissant (1) présente au moins un élément de raccordement (60), lequel est relié à un élément de couplage (22, 24) par au moins un boulon d'accouplement (70), dans lequel l'élément de raccordement (70) est mis en place pour être raccordé mécaniquement à un dispositif de poussée et transmettre une force de compression et/ou de traction (210) du dispositif de poussée à la moitié de cadre (10, 20) présentant l'élément de couplage respectif (22, 24), et **caractérisé en ce que**
le boulon d'accouplement (70) est réalisé avec une longueur telle que l'élément de couplage y étant raccordé (22, 24) et aussi par conséquent la moitié de cadre (10, 20) comprenant cet élément de couplage (22, 24) peuvent être déplacés en translation du fait d'un mouvement relatif entre l'élément de couplage raccordé (22, 24) et le boulon d'accouplement (70) et/ou entre le boulon d'accouplement (70) et l'élément de raccordement (60).

2. Cadre coulissant selon la revendication 1,
**caractérisé en ce**
**que** les articulations (25, 26, 27, 28) des deux moitiés de cadre (10, 20) sont disposées par paire les unes en face des autres, dans lequel les axes de rotation (40) des articulations (25, 26, 27, 28) de la paire d'articulations respective (50) sont disposés pratiquement sur une ligne axiale, tandis que les axes d'articulation (41) et les pivots d'articulation (42) des articulations (25, 26, 27, 28) de la paire d'articulations respective (50) sont séparés les uns des autres.

3. Cadre coulissant selon l'une quelconque des revendications précédentes,
**caractérisé en ce**
**qu'**il comprend au moins un dispositif de compression (100), avec lequel une moitié de cadre (10, 20) peut être sollicitée par au moins une force (120) en direction de l'autre moitié de cadre (10, 20), de sorte qu'un échantillon (140) peut être fixé par adhérence entre les moitiés de cadre (10, 20) moyennant la pression induite par les moitiés de cadre (10, 20).

4. Cadre coulissant selon la revendication 3,
**caractérisé en ce**
**que** le dispositif de compression (100) est constitué par au moins une unité piston-vérin (110).

5. Cadre coulissant selon la revendication 4,
**caractérisé en ce**
**que** le piston de l'unité piston-vérin (110) est réalisé sous la forme d'un piston creux (111) et que le cadre coulissant (1) comprend un outil de positionnement, lequel peut être logé avec précision dans le piston creux (111) de l'unité piston-vérin (110) et peut être inséré dans un trou de positionnement (146) de l'échantillon ou le traverser pour placer l'échantillon (140) dans une position exacte par rapport aux moitiés de cadre (10, 20).

6. Dispositif de contrôle servant à déterminer au moins une propriété d'un échantillon (140) lors de l'action d'une contrainte de poussée, comprenant un cadre coulissant (1) selon l'une quelconque des revendications 2 à 5 ainsi qu'un échantillon (140), qui est conçu sensiblement sous une forme dodécagonale s'étendant dans un plan, dans lequel il présente quatre bords de délimitation extérieurs (141), qui sont pratiquement perpendiculaires à leurs bords de délimitation extérieurs adjacents respectifs (141) et sont séparés par des découpes angulaires (142), dans lequel le sommet (143) d'une découpe angulaire respective (142) se situe sur une diagonale (144), qui relie des points d'intersection diamétralement opposés des droites (145) établies sur les bords de délimitation extérieurs adjacents (141), et les axes de rotation (40) des articulations disposées par paire (25, 26, 27, 28) des moitiés de cadre (10, 20) passent pratiquement par les sommets (143) des découpes angulaires (142).

7. Procédé de détermination d'au moins une propriété d'un échantillon (140) lors de l'action d'une contrainte de poussée, lors duquel un échantillon (140) est enserré dans un cadre coulissant (1) selon l'une quelconque des revendications 1 à 5 et une contrainte de poussée est générée dans l'échantillon (140) moyennant la sollicitation du cadre coulissant 1 par un couple de forces.

8. Procédé de détermination d'au moins une propriété d'un échantillon lors de l'action d'une contrainte de poussée selon la revendication 7,
**caractérisé en ce**
**que** l'échantillon (140) est positionné par rapport aux moitiés de cadre (10, 20) moyennant au moins deux outils de positionnement logés avec précision dans des pistons conçus comme des pistons creux (111) des unités piston-vérin (110), du fait qu'une partie de l'outil de positionnement respectif est insérée avec précision dans un trou de positionnement (146) de l'échantillon (140) et les moitiés de cadre (10, 20) sont ensuite serrées l'une contre l'autre, de sorte que l'échantillon (140) est fixé par adhérence.

9. Procédé de détermination d'au moins une propriété d'un échantillon (140) lors de l'action d'une contrainte de poussée selon l'une quelconque des revendications 7 et 8, **caractérisé en ce**
**qu'**un mouvement relatif en translation est exécuté entre un élément de couplage (22, 24) raccordé à un boulon d'accouplement (70) et le boulon d'accouplement (70) et/ou entre le boulon d'accouplement (70) et un élément de raccordement (60) raccordé au boulon d'accouplement (70) pour faire varier la distance entre les moitiés de cadre (10, 20), afin d'extraire ou d'insérer l'échantillon (140) dans l'espace entre les moitiés de cadre (10, 20).
